# EUROPÄISCHE PATENTSCHRIFT

(11) **EP 3 060 540 B1**
(45) Veröffentlichungstag und Bekanntmachung des Hinweises auf die Patenterteilung: **27.06.2018**
(21) Anmeldenummer: 14786869.9
(22) Anmeldetag: 20.10.2014
(51) Int. Cl.: C07C 49/167, C07C 45/67

(54) **VERFAHREN ZUR HERSTELLUNG VON HALOGENKETONEN**
METHOD FOR MANUFACTURING HALOGEN KETONES
PROCÉDÉ DESTINÉ À LA FABRICATION DE CÉTONES HALOGÈNES

(30) Priorität: 23.10.2013 EP 13189817
(43) Veröffentlichungstag der Anmeldung: 31.08.2016
(73) Patentinhaber: Bayer CropScience Aktiengesellschaft, 40789 Monheim am Rhein (DE)
(72) Erfinder: PAZENOK, Sergii, 42699 Solingen (DE); FUNKE, Christian, 42799 Leichlingen (DE)
(74) Vertreter: BIP Patents
(86) Internationale Anmeldenummer: PCT/EP2014/072388
(87) Internationale Veröffentlichungsnummer: WO 2015/059067

(56) Entgegenhaltungen:
- EP-A1- 0 623 575
- DE-A1- 4 313 794

## Beschreibung

Die vorliegende Erfindung betrifft ein neues Verfahren zur Herstellung von Halogenketonen.

Halogenketone, wie z.B. Difluoraceton und Trifluoraceton, sind wichtige Zwischenstufen bei der Herstellung von biologisch aktiven Verbindungen: WO 2009/000442.

Difluoraceton oder Trifluoraceton können z. B. aus Difluoracetat oder Trifluoressigsäure und Methylmagnesiumbromid hergestellt werden (Isr. Journal of Chemistry, 1999, 39, 155). Allerdings beträgt die Ausbeute für Difluoraceton gerade einmal 47 % und für Trifluoraceton 56 %.

Di- und Trifluoracetone können auch durch Spaltung von Trifluoracetoacetat in Anwesenheit von zwanzigprozentiger Schwefelsäure hergestellt werden (Tetrahedron, 1964, 20, 2163). Der Nachteil dieser Reaktion ist, dass es schwierig ist, einen geeigneten Werkstoff zu finden, der nicht korrodiert und indem die Reaktion durchgeführt werden kann. Dabei ist nicht nur die Schwefelsäure die Korrosion verursachende Komponente, sondern auch das freigesetzte Fluorid. Diese Kombination macht die Anwendung von Stahlemaille, Edelstahl, sowie Hastelloy Kesseln nicht möglich.

EP0623575 (B1) beschreibt die Synthese von Ketonen durch Umsetzung einer Carbonsäure mit einem Ketoester in Anwesenheit einer katalytischen Menge eines Onium Salzes:

CF₃COOH + CF₃C(O)CH(COOEt) + CH₃SO₃H →CF₃COCH₃ + 2CF₃COOEt

Dieses Verfahren ist nicht wirtschaftlich: Es wird zusätzlich eine Carbonsäure wie CF₃COOH benötigt. Außerdem entsteht ein Ester, der Probleme bei der Reinigung des Produktes verursacht.

Im Lichte des zuvor beschriebenen Standes der Technik liegt der vorliegenden Erfindung die Aufgabe zugrunde, ein Verfahren zur Verfügung zu stellen, dass die zuvor genannten Nachteile nicht aufweist und somit einen Zugang zu Halogenketonen in hohen Ausbeuten gewährt.

Die zuvor beschriebene Aufgabe wurde gelöst durch ein neues Verfahren zur Herstellung von Halogenketonen der Formel (I)

R¹COCH₃ (I),

in welcher
- R¹: für Halogenalkyl steht,
dadurch gekennzeichnet, dass Ketoester der Formel (II)

R¹COCH₂COOR² (II),

in welcher
R² für Alkyl oder Benzyl steht und
R¹ wie oben definiert ist,
in Gegenwahrt von Phosphorsäure gemäß folgendem Schema gespalten werden.

R¹COCH₂COOR²+H₃PO₄ →R¹COCH₃+CO₂+R²OH

Überraschenderweise lassen sich die Halogenketone der Formel (**I**) unter den erfindungsgemäßen Bedingungen mit guten Ausbeuten und in hoher Reinheit herstellen, ohne stark korrosive Reaktionsbedingungen zu haben, womit das erfindungsgemäße Verfahren die oben genannten Nachteile der im Stand der Technik vorbeschriebenen Herstellungsverfahren überwindet.

*Bevorzugt* ist ein erfindungsgemäßer Prozess, bei dem die Restedefinitionen der Verbindungen der Formeln (I) und (II) wie folgt sind:
- R¹: wird ausgewählt aus CF₃, CF₂H, CF₂Cl;
- R²: wird ausgewählt aus Methyl, Ethyl, n-Propyl, Benzyl.

*Besonders bevorzugt* ist ein erfindungsgemäßer Prozess, bei dem die Restedefinitionen der Verbindungen der Formeln (I) und (II) wie folgt sind:
- R¹: ist CF₂H;
- R²: wird ausgewählt aus Methyl, Ethyl.

### Allgemeine Definitionen

**Halogenalkyl:** geradkettige oder verzweigte Alkylgruppen mit 1 bis 6 und bevorzugt 1 bis 3 Kohlenstoffatomen , wobei in diesen Gruppen teilweise oder vollständig die Wasserstoffatome durch Halogenatome wie vorstehend genannt ersetzt sein können, z.B. (aber nicht beschränkt auf) C₁-C₃-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor,2-difluorethyl, 2,2-Dichlor-2-fluorethyl, 2,2,2-Trichlorethyl, Pentafluorethyl und 1,1,1-Trifluorprop-2-yl.

**Alkyl**-Gruppen sind im Zusammenhang mit der vorliegenden Erfindung lineare, verzweigte oder ringförmige gesättigte Kohlenwasserstoff-Gruppen. Die Definition C₁-C₁₂-Alkyl umfasst den größten hierin definierten Bereich für einen Alkyl-Gruppe. Im Einzelnen umfasst diese Definition beispielsweise die Bedeutungen Methyl, Ethyl, n-, iso-Propyl, n-, iso-, sec- und t-Butyl, n-Pentyl, n-Hexyl, 1,3-Dimethylbutyl, 3,3-Dimethylbutyl, n-Heptyl, n-Nonyl, n-Decyl, n-Undecyl oder n-Dodecyl.

### Verfahrensbeschreibung

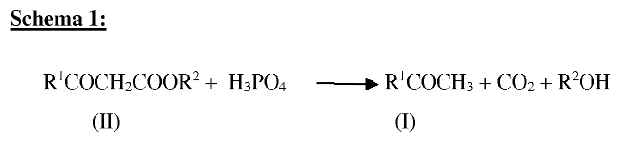

Die als Ausgangsverbindungen dienenden Ketoesterverbindungen der Formel (II) sind bekannt und kommerziell erhältlich. Die Temperatur bei der erfindungsgemäßen Reaktion liegt im Bereich von 20 bis 200°C, vorzugsweise im Bereich von 70 bis 130°C.

Das erfindungsgemäße Verfahren kann gegebenenfalls kontinuierlich betrieben werden.

Die Menge H₃PO₄ beträgt zwischen 5 bis 500 g für 1 Mol der Verbindung der Formel (I), bevorzugt 20-350 g. Nach der Isolierung des Produktes wird das H₃PO₄ ohne Aufreinigung wieder eingesetzt. Mindestens 5 solcher Zyklen sind ohne Verlust an Ausbeute möglich. Es wird bevorzugt wässrige Lösung von H₃PO₄ eingesetzt. Die Konzentration der H₃PO₄ beträgt 20-85%, vorzugsweise 85 %. Vorzugsweise wird technische Ware mit einem Gehalt von 70-85 % verwendet. Es ist auch möglich Polyphosphorsäure einzusetzen. Gegebenenfalls wird die Reaktion in einem inerten Lösungsmittel wie Chlorbenzol, Toluol durchgeführt. Die Korrosion von Glas und Metallwerkstoffen wurde bei diesem Verfahren nicht beobachtet.

### Beispiel 1

### Difluoraceton HCF₂COCH₃

In einem 250 ml Mehrhalskolben mit aufgesetzter Destillationsbrücke wurden 150 ml H₃PO₄ (w.w. % 85) vorgelegt und auf 100 bis 105°C erhitzt. 132 g Ethyldifluoracetoacetat mit einer Reinheit von w.w. % 91 (0.720 mol) wurden über eine Spritzpumpe innerhalb von 3 Stunden hinzugefügt. Das Destillat mit einem Siedepunkt von 40-60°C wurde binnen 5 Stunden kontinuierlich abgenommen. Nach 5 Stunden ist die Reaktion beendet. Insgesamt wurden 75 g klare farblose Flüssigkeit gesammelt.

Die Zusammensetzung der Fraktion (bestimmt durch ¹⁹F NMR) :
HCF₂COCH₃ w.w. % 70.
HCF₂C(OH)₂CH₃ w.w. % 11. Hydrat
HCF₂C(OEt)(OH)CH₃ w.w. % 14. Monoketal

Das Gemisch kann ohne Reinigung weiter eingesetzt werden. Gegebenenfalls kann eine zweite Destillation über H₃PO₄ zur weiteren Reinigung durchgeführt werden. Gleichzeitig werden Hydrat und Monoketal gespalten und ins Difluoraceton überführt.

Beim Einsatz von 5 g H₃PO₄ nach Destillation erhält man 67 g Difluoraceton (HCF₂COCH₃) mit einer Reinheit von 95 bis 96 %. Die Ausbeute beträgt 94 bis 95 %.

## Patentansprüche

1. Verfahren zur Herstellung von Halogenketonen der Formel (I)
R¹COCH₃ (I),
in welcher
R¹ für Halogenalkyl steht,
**dadurch gekennzeichnet, dass** Ketoester der Formel (II)
R¹COCH₂COOR² (II),
in welcher
R² für Alkyl umfassend lineare, verzweigte oder ringförmige gesättigte Kohlenwasserstoff-Gruppen oder Benzyl steht und R¹ wie oben definiert ist,
in Gegenwahrt von Phosphorsäure gemäß dem folgenden Schema gespalten werden:

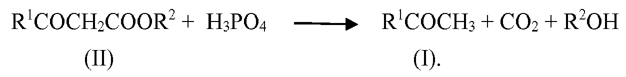

2. Verfahren gemäß Anspruch 1, **dadurch gekennzeichnet, dass**
R¹ ausgewählt wird aus CF₃, CF₂H, CF₂Cl;
R² ausgewählt wird aus Methyl, Ethyl, n-Propyl, Benzyl.

3. Verfahren gemäß Anspruch 1, **dadurch gekennzeichnet, dass**
R¹ CF₂H ist;
R² ausgewählt wird aus Methyl, Ethyl.

4. Verfahren nach einem der Ansprüche 1 bis 3 **dadurch gekennzeichnet, dass** es bei einer Temperatur von 70 bis 130°C durchgeführt wird.

5. Verfahren nach einem der Ansprüche 1 bis 4 **dadurch gekennzeichnet, dass** es kontinuierlich durchgeführt wird.

## Claims

1. Method for producing haloketones of formula (I)
R¹COCH₃ (I),
where
R¹ is haloalkyl,
**characterized in that** ketoesters of formula (II)
R¹COCH₂COOR² (II),
where
R² is alkyl, comprising linear, branched or cyclic saturated hydrocarbon groups, or benzyl and
R¹ is as defined above,
are cleaved in the presence of phosphoric acid according to the following scheme:

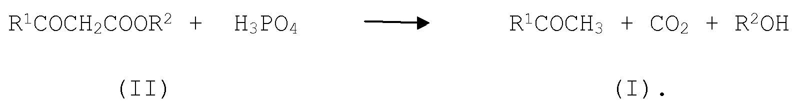

2. Method according to Claim 1, **characterized in that**
R¹ is selected from CF₃, CF₂H, CF₂Cl;
R² is selected from methyl, ethyl, n-propyl, benzyl.

3. Method according to Claim 1, **characterized in that**
R¹ is CF₂H;
R² is selected from methyl, ethyl.

4. Method according to any one of Claims 1 to 3, **characterized in that** it is carried out at a temperature of from 70°C to 130°C.

5. Method according to any one of Claims 1 to 4, **characterized in that** it is carried out as a continuous operation.

## Revendications

1. Procédé de fabrication d'halogénocétones de formule (I)
R¹COCH₃ (I)
dans laquelle
R¹ représente halogénoalkyle,
**caractérisé en ce que** des cétoesters de formule (II)
R¹COCH₂COOR² (II)
dans laquelle
R² représente alkyl comprenant des groupes hydrocarbonés saturés linéaire, ramifiés ou cycliques, ou benzyle, et
R¹ est tel que défini précédemment,
sont clivés en présence d'acide phosphorique selon le schéma suivant :

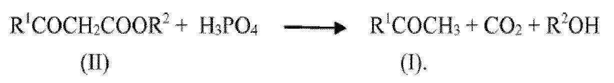

2. Procédé selon la revendication 1, **caractérisé en ce que**
R¹ est choisi parmi CF₃, CF₂H, CF₂Cl ;
R² est choisi parmi méthyle, éthyle, n-propyle, benzyle.

3. Procédé selon la revendication 1, **caractérisé en ce que**
R¹ représente CF₂H ;
R² est choisi parmi méthyle, éthyle.

4. Procédé selon l'une quelconque des revendications 1 à 3, **caractérisé en ce qu'**il est réalisé à une température de 70 à 130 °C.

5. Procédé selon l'une quelconque des revendications 1 à 4, **caractérisé en ce qu'**il est réalisé en continu.
